# EUROPEAN PATENT SPECIFICATION

(11) **EP 3 543 495 B1**
(45) Date of publication and mention of the grant of the patent: **21.10.2020**
(21) Application number: 19164417.8
(22) Date of filing: 21.03.2019
(51) Int. Cl.: F01N 3/20, F01N 9/00, F01N 11/00, F01N 13/00, G01N 27/00, G01N 33/00, G01M 15/00, G01M 15/10

(54) **ANALYSIS METHOD OF A SIGNAL GENERATED BY A NOX SENSOR OF AN ACTIVE SCR CATALYST AND CORRESPONDING METHOD AND SYSTEM FOR CONTROLLING THE ACTIVE SCR**
ANALYSEVERFAHREN EINES SIGNALS GENERIERT DURCH EINEN NOX-SENSOR EINES AKTIVEN SCR-KATALYSATORS UND KORRESPONDIERENDES VERFAHREN UND SYSTEM ZUR STEUERUNG DES AKTIVEN SCR
PROCÉDÉ D'ANALYSE D'UN SIGNAL GÉNÉRÉ PAR UN CAPTEUR NOX D'UN CATALYSEUR SCR ACTIF ET PROCÉDÉ ET SYSTÈME CORRESPONDANTS POUR CONTRÔLER LE SCR ACTIF

(30) Priority: 21.03.2018 IT 201800003831
(43) Date of publication of application: 25.09.2019
(73) Proprietor: FPT Motorenforschung AG, 9320 Arbon (CH)
(72) Inventor: GIANELLA, Matteo, 9000 ST. GALLEN (CH); ABT, Michael, 9500 WIL (CH)
(74) Representative: Bongiovanni, Simone

(56) References cited:
- WO-A1-2015/095332
- DE-A1-102013 205 814
- DE-A1-102015 206 120
- US-A1- 2011 252 767
- US-A1- 2012 085 083
- US-A1- 2015 276 698
- CHIH CHENG CHOU ET AL: "Interpretation of the Oscillating Signals of the Smart NOx Sensors Used in Urea Selective Catalyst Reduction Systems via Spectral Analysis", APPLIED MECHANICS AND MATERIALS, vol. 479-480, 1 January 2014 (2014-01-01), pages 719-723, XP009506881, DOI: 10.4028/www.scientific.net/AMM.479-480.719

## Description

### Field of application of the invention

The present invention refers to an analysis method according to the preamble of claim 1, which corresponds to US2015-276698A1, in the field of exhaust gas after-treatment systems of internal combustion engines, and more precisely to methods and systems for controlling an active SCR which composes an exhaust gas after-treatment system (ATS).

### State of the art

Exhaust gas after-treatment systems, especially for diesel cycle engines, are provided with an active SCR (Selective Catalytic Reduction), i.e. provided with a relative metering unit of a urea-based reducing agent arranged upstream of said SCR in relation to a direction of circulation of exhaust gas in an exhaust line of an internal combustion engine.

In the following description, by SCR we mean an "active SCR", therefore provided with a relative metering unit of urea-based reducing agent.

Immediately downstream of the SCR a NOx sensor is normally arranged and for this reason said NOx sensor is called "NOx sensor of the SCR" also known as tailpipe NOx sensor.

The urea is hydrolysed into ammonia (NH3) and stored in the SCR. The NOx produced by the internal combustion engine are reduced to N2 due to the presence of ammonia stored in the SCR.

The NOx sensor of the SCR allows appropriate regulation of the injection of urea-based reducing agent, minimizing the NOx measured downstream of the SCR.

In order to avoid releasing excess ammonia into the atmosphere, an NH3 sensor is generally also implemented downstream of the SCR.

The presence of the NH3 sensor allows not only correct monitoring of the excess NH3 but also correction of the readings of the NOx sensor of the SCR.

In fact, it is known to a person skilled in the art that the NOx sensor is affected by a cross-sensitivity to ammonia.

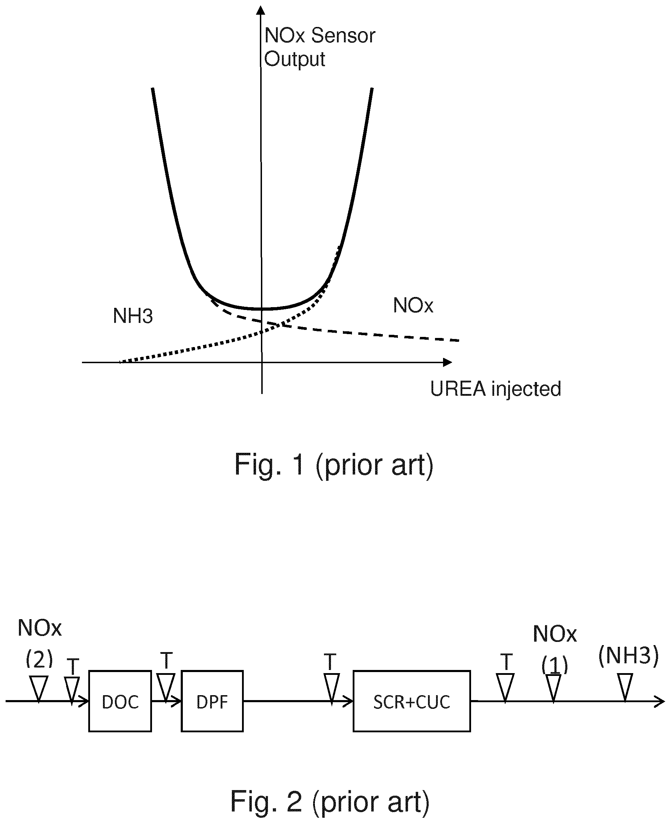

A schematic example of a characteristic curve of an NOx sensor is shown in figure 1.

It can be clearly seen that this characteristic is an even function and therefore there is an ambiguity on the reading, such that two opposite operating conditions of the degree of storage of ammonia in the SCR correspond to the same sensor output signal value.

The elimination of the NH3 sensor has been attempted several times, for example by means of the method described in EP2339136 according to which only one NOx sensor is implemented arranged downstream of the SCR.

The ammonia emissions can be modelled to compensate the inefficiency of the NOx sensor. Unfortunately, however, said models are not reliable in unpredictable conditions, such as transients.

According to other known methods, a variation of the signal generated by the NOx sensor is related to a variation in the injection of urea-based reducing agent.

When the two variations have the same sign, this implies that the signal generated by the NOx sensor is representative of the NH3 released by the SCR rather than by the residual NOx and therefore corrective recovery actions are activated.

Said strategies, however, are impracticable since the dynamics of the hydrolysis process are very slow. This therefore entails introduction of non-converted NOx or ammonia in excess into the environment until the method detects the real operating conditions of the SCR.

Figure 2 of the known art shows an example of exhaust gas after-treatment system (ATS) of a diesel cycle internal combustion engine according to the known art.

T indicates a temperature sensor. NOx indicates a NOx sensor and NH3 indicates an ammonia sensor.

A first NOx sensor (1) coincides with the above-mentioned NOx sensor of the SCR also called tailpipe, while a second NOx sensor (2) is arranged upstream of the ATS and therefore is indicated as "engine NOx sensor" since it is arranged immediately downstream of the internal combustion engine exhaust manifolds and upstream of the after-treatment system. Therefore, it measures the NOx produced by the engine prior to any treatment of the relative exhaust gases.

The DOC (Diesel Oxidation Catalyst) components, the DPF (Diesel Particulate Filter), and the SCR are components known to the person skilled in the art. The arrows, furthermore, indicate the direction of outflow of the exhaust gases, produced by the internal combustion engine, through the ATS.

The CUC (Clean Up Catalyst) component is optional and is implemented in an attempt to abate the excess ammonia. It too is known to a person skilled in the art.

### Summary of the invention

The aim of the present invention is to propose an analysis method of a signal produced by a NOx sensor arranged downstream of an active SCR such as to avoid the implementation of an NH3 sensor downstream of said SCR without running into the problems of the known art.

Said analysis allows the above-mentioned ambiguity of the NOx sensor to be solved without acting on the injection of urea-based reducing agent.

The basic idea of the present invention is to analyse the signal generated by the NOx sensor by isolating a predetermined first frequency band thereof. When an intensity value of the portion of signal in said frequency band exceeds a first predetermined threshold, then said signal is deemed indicative of a measurement of NOx; when, on the other hand, said value is lower than a second predetermined threshold, then said signal is deemed indicative of a measurement of NH3.

The first predetermined threshold is greater than or equal to the second predetermined threshold.

Said operation is conducted without in any way acting on the control of the SCR urea metering unit.

Furthermore, said predetermined frequency band excludes the continuous component of the signal generated by the NOx sensor.

When said signal is indicative of a measurement of NH3, corrective actions are taken, for example, known per sé, which aim at restoring a correct storage of ammonia in the SCR. Preferably, an injection of urea-based reducing agent of the active SCR is drastically reduced.

The above-mentioned first frequency band can be appropriately varied in relation to the internal combustion engine operating conditions. In other words, both the amplitude and the median frequency thereof can be appropriately varied.

For said purpose the frequency band can be selected on the basis of measurements taken via a second NOx sensor arranged on the exhaust gas after-treatment system defined by said active SCR and arranged upstream of said active SCR. In particular, the second signal generated by said second NOx sensor is analysed in the frequency domain and a second non-continuous frequency band is isolated having amplitude greater than all the other bands.

Consequently, said first band is forced to coincide with said second band.

The exclusion of the continuous component of the signal can be achieved by means of high-pass filtering.

According to a preferred variation of the present invention, when the operating conditions of the internal combustion engine are such that the harmonic components of said second signal are excessively weak, i.e. no frequency band has an amplitude exceeding a second predetermined threshold, then an operating parameter of the internal combustion engine is varied so as to increase the harmonic components of said second signal and consequently of the first signal.

Obviously the present analysis does not impact on the adjustment scheme of the urea-based reducing agent metering unit.

The present analysis operates essentially on the signals generated by NOx sensors; only when this is not sufficient does it intervene on an operating parameter which affects the relative production of NOx. In particular, said parameter is varied so as to maintain unchanged the level of NOx produced on average by the internal combustion engine, but it increases relative ripples thereof in the time domain implying a greater spectral dispersion in the frequency domain.

A variation of the production of NOx does not necessarily affect control of the metering of urea-based reducing agent. In fact, the SCR behaves like a capacity and therefore any ripples can be partly or wholly compensated by the NH3 storage, as long as the mean remains unchanged.

The claims describe preferred variations of the invention, forming an integral part of the present description.

### Brief description of the figures

Further objects and advantages of the present invention will become clear from the following detailed description of an embodiment example thereof (and variations thereof) and from the accompanying drawings provided purely for illustrative non-limiting purposes, in which:
Figure 1 shows the characteristic of a NOx sensor according to the known art;
Figure 2 shows a device for the abatement of contaminants produced by an internal combustion engine provided with two NOx sensors having the characteristic of figure 1;
Figure 3 shows a flow diagram representing a preferred variation of the method subject of the present invention;
Figure 4 shows a further flow diagram of optional procedures of the invention;
Figure 5 shows a diagram of an internal combustion engine provided with an exhaust gas after-treatment system and a control system subject of the present invention. When the flow diagram blocks of figures 3 and 4 are shown in broken lines, they represent corresponding optional steps.

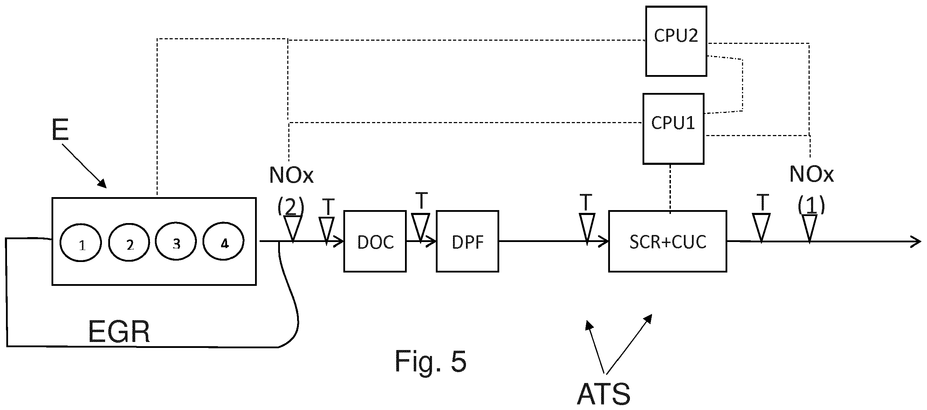

In figure 5, the broken lines indicate analog or digital electrical connections.

The same reference numbers and letters in the figures identify the same elements or components.

In the context of the present description the term "second" component does not imply the presence of a "first" component. Said terms are in fact used only for clarity and should not be understood in a limiting manner.

### Detailed disclosure of embodiment examples

Figure 5 shows schematically an internal combustion engine E provided with any number of cylinders, for example four from 1 to 4.

An exhaust gas treatment system is connected to the exhaust manifold of the internal combustion engine E.

The last component of the ATS is the above-mentioned active SCR, i.e. provided with a metering unit for metering urea-based reducing agent.

The NOx sensor indicated by NOx(1) is arranged downstream of the active SCR.

A processing unit CPU1 controls the injection of urea-based reducing agent on the basis of various parameters including NOx measurements carried out by means of said first NOx sensor NOx(1), both by means of a second NOx sensor NOx(2) arranged upstream of the SCR and preferably immediately downstream of the exhaust manifold of the internal combustion engine E, and on the basis of operating parameters of the internal combustion engine including:
- Injection times of the fuel into the cylinders,
- Opening angles of the intake and/or exhaust valves,
- Ignition advances when the internal combustion engine E is of the spark-ignition type,
- Percentage of recirculated exhaust gases, when the engine E is provided with an EGR device, i.e. a device adapted to recirculate exhaust gases produced by said internal combustion engine to the intake manifold, so as to adjust the combustion temperatures and therefore the level of NOx produced by the same.

The analysis method, subject of the present invention, provides for
- a first step 1 of acquiring the electrical signal, which can be analog or digitalized by a sensor comprising an A/D interface and preferably implementing a CAN protocol, generated by the first NOx sensor NOx(1), consequently leading to
- a second step 2 of analysing the signal generated by the NOx sensor isolating a predetermined first frequency band and calculating a relative intensity value H, consequently leading to
- a third step CK1 of comparing said intensity value with a first predetermined threshold Th1, i.e. if H >= Th1,
then said signal is deemed indicative of a measurement of NOx when said value is greater than or equal to said first predetermined threshold.

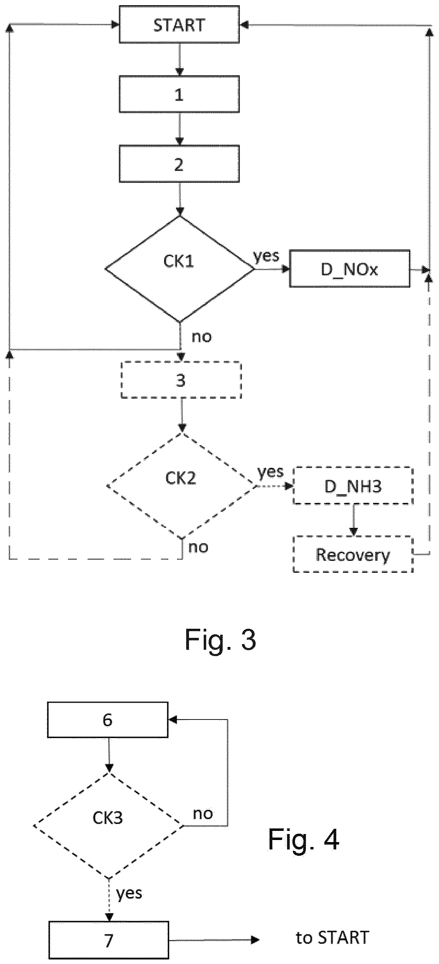

Figure 3 shows an illustrative flow diagram with the START initialization step and the following steps described above.

It is obviously a cyclic diagram, always beginning again from START.

The method could terminate without positively encountering the opposite case, i.e. the case in which the signal is indicative of a measurement of NH3.

According to the invention, when the intensity value is lower(H < Th1) than said first predetermined threshold, the method comprises a fifth consequent step (CK2) of comparing said value with a second predetermined threshold Th2, lower than or equal to said first predetermined threshold Th1, then said signal is deemed indicative of a measurement of NH3 when said value is lower than said one second predetermined threshold H < Th2.

When Th1 and Th2 are equal, obviously, identification of the condition of slippage, i.e. excess of NH3 measured, is implicitly identified already at the first comparison CK1. However, the use of a second threshold Th2 lower than Th1 is preferred in order to be certain of the analysis result. This is particularly useful when it is possible to force an increase of the intensity of said band, as described below. Many methods can be used to isolate the above-mentioned frequency band and calculate the amplitude thereof. Preferably the Fast Fourier Transform is used, therefore operating in the frequency domain. Nevertheless, it is possible to operate in the time domain, with a band-pass filter and measurement of a mean intensity of the signal by means of an integrator circuit. Another preferred variation of the invention exploits calculation of the orthogonal correlation between the signal of the first sensor NOx(1) and a sinusoid rippling at a given frequency, central with respect to the frequency band of interest. This results in an evaluation of the ripple amplitude of the signal NOx(1) around said frequency through said correlation.

In any case, the above options are obvious for a person skilled in the art.

As described above, generally, a second NOx sensor is arranged upstream of the SCR. This is therefore able to measure the level of the NOx produced by the engine prior to the abatement thereof and therefore is also able to more easily identify the relative time ripples which define spectral components in the frequency domain.

According to a preferred variation of the method, a sixth step 6 is provided for analysing a second signal generated by said second sensor NOx(2) in the same first frequency band in order to verify that said frequency band has a sufficient energy content for the analysis on the first sensor NOx(1) to be considered reliable. If said amplitude is greater than a third predetermined threshold Th3, the analysis of the signal of the first sensor NOx(1) is enabled and the result thereof is used in the subsequent steps, otherwise the strategy can be temporarily suspended or a third step 3 is carried out which consists in forcing said internal combustion engine to produce NOx emissions rippling in time. While the internal combustion engine is thus forced to emit a level of NOx rippling in time, the present analysis strategy can be resumed.

In a further embodiment variation, the second sensor NOx(2) can be used in order to vary the frequency band in which the signal of the first sensor NOx(1) is analysed. For this purpose, the signal of the sensor NOx(2) will be analysed in the frequency domain, selecting a second frequency band having greater intensity.

Obviously the continuous component which is not significant for the present purposes is discarded and therefore the band is by definition "harmonic", since the continuous component is excluded. The band is selected that expresses the greatest relative intensity among the most significant bands in terms of intensity.

Therefore, for the purposes of the present invention, it may be advantageous, especially if operating in the time domain, to high-pass filter said signal generated by said NOx sensor and/or said signal generated by said second NOx sensor.

Subsequently, the method provides for a seventh step 7 of forcing said first band to coincide with said second frequency band.

In other words, the band analysed on the signal generated by the first sensor is obtained from analysis of the second sensor.

The content of what has just been described is shown in figure 4. The result of the flow diagram of figure 4 operates on the START initialization step of the flow diagram of figure 3.

The method preferably comprises an eighth step CK3 of verifying that said intensity of said second band exceeds a third predetermined threshold Th3, and if positive CK3=yes said seventh step is carried out, otherwise said previously set first predetermined band is left unchanged.

In other words, if the second band identified through the signal generated by the second sensor does not express a significant intensity, there is no point in modifying the frequency band subject of the analysis of the signal generated by the first sensor. Rather, the method provides for the above-mentioned third step 3 of forcing said internal combustion engine to produce emissions of NOx rippling in time, thus the present strategy can be implemented also when the internal combustion engine is operating in stationary conditions in terms of NOx production.

Graphically, the third step 3 is shown by a broken line between the first check CK1 and the second check CK2 which is optional. Step 3 can be adopted independently of adoption of the second check CK2.

When the situation is uncertain, i.e. the intensity of the signal in the first band is between Th1 and Th2, then it is preferable to force said internal combustion engine to produce NOx emissions rippling in time.

Obviously, if not even this strategy induces an increase in the intensity value of the first signal in said first predetermined band, then it is more probable that the sensor is measuring NH3 rather than NOx.

The method could simply be an analysis method and not necessarily a control method. When the present analysis method is part of a control method, then, when the intensity value is still below Th1 following the operation of forcing the internal combustion engine to produce a level of NOx rippling in time, the method could provide for skipping the second check CK2 directly performing a recovery procedure.

Said recovery procedure can comprise a sudden reduction in injection of a urea-based reducing agent into said active SCR and/or an increase in production of NOx by the internal combustion engine to consume the excess of hydrolysed NH3.

The method of the present invention can be performed, for example, by means of a second processing unit CPU2. This can be limited only to the content of the flow diagram of figure 3 when it is configured to acquire only the signal generated by the first sensor NO(1) or can also perform the steps of the flow diagram of figure 4 when it is configured to acquire also the signal generated by the second sensor NO(2).

The invention also concerns an internal combustion engine comprising
- an exhaust gas after-treatment system (ATS) comprising an active SCR formed of a SCR and a metering unit for metering urea-based reducing agent arranged upstream of the SCR according to an outflow direction of the exhaust gases produced by the internal combustion engine,
- a first NOx sensor arranged downstream of said active SCR,
- a first processing unit CPU1 configured to control an injection of said urea-based reducing agent on the basis of measurement of NOx performed by said first NOx sensor,
- a second processing unit CPU2 configured to perform the analysis method subject of the present invention.

The second processing unit CPU2 is preferably configured to communicate to said first processing unit whether said signal generated by said first sensor is considered indicative of a measurement of NOx and/or of NH3.

In fact, generally, the first processing unit performs the recovery operations.

More preferably, the present invention is performed by the same processing unit configured to control the metering of urea-based reducing agent.

More preferably, said processing unit is the same one configured to control said internal combustion engine. It is generally called ECU (Engine Control Unit).

As regards the procedure of forcing the internal combustion engine to emit a level or flow of NOx rippling in time, this can be obtained in different ways.

For example by differentiating an operating parameter of a subset of cylinders. For example, by causing some of them to burn leaner and the others to burn relatively richer.

Or it is possible to act on the EGR valve, forcing a ripple thereof in closing around a pre-calculated position.

Furthermore, the control strategies that impact on the production of NOx can be conflicting, for example, it can be forseen to lower the mean emission over time, which would favour a pronounced ripple.

The present invention can be advantageously achieved by means of a computer program that comprises coding means for the realization of one or more steps of the method, when this program is run on a computer. Therefore it is understood that the protective scope extends to said computer program and furthermore to computer-readable means which comprise a recorded message, said computer-readable means comprising program coding means for the realization of one or more steps of the method, when said program is run on a computer.

Variations on the non-limiting example described are possible, without departing from the protective scope of the present invention, comprising all the embodiments equivalent for a person skilled in the art.

From the above description, a person skilled in the art is able to produce the subject of the invention without introducing further construction details. The elements and characteristics illustrated in the various preferred embodiments, including the drawings, can be combined without departing from the protective scope of the present application. What is described in the chapter relative to the state of the art serves only for a better understanding of the invention and does not represent a declaration of existence of what is described. Furthermore, if not specifically excluded in the detailed disclosure, what is described in the state of the art chapter shall be considered as integral parts of the detailed disclosure.

## Claims

1. An analysis method of a signal generated by a NOx sensor arranged downstream of an active SCR catalyst, the method comprising:
- a first step of acquiring (1) the signal generated by the NOx sensor,
- a second consequent step (2) comprising:
a) isolating a predetermined first frequency band of said signal, and
b) calculating an intensity value (H) of said signal in said first frequency band,
**characterized by** comprising a third consequent step (CK1) of comparing said intensity value with a first predetermined threshold (Th1); said signal being deemed indicative of a measurement of NOx when said intensity value is greater than or equal (H>=Th1) to said first predetermined threshold; wherein, when said intensity value is lower (H<Th1) than said first predetermined threshold, the method comprises a fifth consequent step (CK2) of comparing said intensity value with a second predetermined threshold (Th2), lower than or equal to said first predetermined threshold (Th1); said signal being deemed indicative of a measurement of NH3 when said intensity value is lower than said second predetermined threshold (H < Th2).

2. The method according to claim 1, wherein said active SCR is included in an exhaust gas after-treatment system (ATS) comprising a second NOx sensor arranged upstream of said active SCR, the method comprising a sixth step of analysing a second signal generated by said second NOx sensor (NOx(2)) in the frequency domain, to
a) isolate said first predetermined frequency band of said second signal and check that a second intensity (H') of said second signal, exceeds a third predetermined threshold (Th3) and/or
b) select a second harmonic frequency band having greatest relative intensity, named second intensity (H'), and a seventh step of forcing said first band to coincide with said second frequency band.

3. The method according to claim 2, further comprising an eighth step (CK3) of checking that said second intensity (H') of said second band exceeds a third predetermined threshold (Th3), and if so (CK3=yes), said seventh step is performed, otherwise said first predetermined band previously set is left unchanged.

4. The method according to claim 3, wherein said active SCR catalyst is included in an exhaust gas after-treatment system (ATS) of an internal combustion engine, and wherein, when said second intensity (H') is lower than said third predetermined threshold (Th3), the method provides for a third step (3) of forcing said internal combustion engine to produce NOx emissions rippling in time.

5. The method according to any one of the preceding claims, wherein said active SCR catalyst is included in an exhaust gas after-treatment system (ATS) of an internal combustion engine, and wherein, when said intensity value (H) is lower than said first predetermined threshold, the method provides for a third step (3) of forcing said internal combustion engine to produce NOx emissions rippling in time.

6. The method according to claim 4 or 5, wherein said signal is deemed indicative of a measurement of NH3 when, following said third step, said intensity value of said first frequency band is lower than said second predetermined threshold.

7. An active SCR control method comprising a first step of performing all the steps of any one of the preceding claims from 1 to 6 and further comprising a further step (Recovery) of performing a recovery procedure when said NOx sensor signal is deemed indicative of a measurement of NH3.

8. The method according to claim 7, wherein said recovery procedure comprises a sudden reduction in injection of a urea-based reducing agent into said active SCR and/or an increase in NOx production by the internal combustion engine.

9. The method according to any one of the preceding claims, wherein said signal generated by said NOx sensor and/or said signal generated by said second NOx sensor is preliminarily high-pass filtered.

10. An active SCR control system comprising a NOx sensor arranged downstream of an active SCR, in relation to an outflow of exhaust gases passing through said active SCR, and a processing unit configured at least to perform all the steps of any one of the claims 1, 4 - 9.

11. An internal combustion engine comprising
- an exhaust gas after-treatment system (ATS) comprising an active SCR formed of a SCR and a metering unit for metering urea-based reducing agent arranged upstream of the SCR, according to an outflow direction of the exhaust gases produced by the internal combustion engine,
- a first NOx sensor arranged downstream of said active SCR,
- a first processing unit (CPU1) configured to control an injection of said urea-based reducing agent on the basis of a measurement of NOx performed by said first NOx sensor,
- a second processing unit (CPU2) configured to perform all the steps of any one of the claims 1, 4 - 9.

12. The engine according to claim 11, wherein said second processing unit is configured to communicate to said first processing unit whether said signal generated by said first sensor is deemed indicative of a measurement of NOx and/or of NH3.

13. The engine according to one of the claims 11 or 12, wherein said ATS is further provided with a second NOx sensor arranged upstream of said active SCR, and wherein said second processing unit is configured to further perform also all the steps of one of the claims 3 or 4.

## Patentansprüche

1. Analyseverfahren eines Signals, das durch einen NOₓ-Sensor erzeugt wird, der stromabwärts eines aktiven SCR-Katalysators angeordnet ist, wobei das Verfahren umfasst:
- einen ersten Schritt des Erfassens (1) des durch den NOₓ-Sensor erzeugten Signals,
- einen zweiten, folgenden Schritt (2), der umfasst:
a) Isolieren eines vorgegebenen ersten Frequenzbandes des Signals und
b) Berechnen eines Intensitätswerts (H) des Signals in dem ersten Frequenzband,
**gekennzeichnet durch** einen dritten, folgenden Schritt (CK1) des Vergleichens des Intensitätswerts mit einem ersten vorgegebenen Schwellenwert (Th1); wobei angenommen wird, dass das Signal einen Messwert von NOₓ angibt, wenn der Intensitätswert größer oder gleich dem ersten vorgegebenen Schwellenwert ist (H ≥ Th1); wobei dann, wenn der Intensitätswert kleiner als der erste vorgegebene Schwellenwert ist (H < Th1), das Verfahren einen fünften, folgenden Schritt (CK2) des Vergleichens des Intensitätswerts mit einem zweiten vorgegebenen Schwellenwert (Th2), der kleiner oder gleich dem ersten vorgegebenen Schwellenwert (Th1) ist, umfasst; wobei angenommen wird, dass das Signal einen Messwert des NH3 angibt, wenn der Intensitätswert kleiner als der zweite vorgegebene Schwellenwert ist (H < Th2).

2. Verfahren nach Anspruch 1, wobei der aktive SCR in einem Abgasnachbehandlungssystem (ATS) enthalten ist, das einen zweiten NOₓ-Sensor umfasst, der stromaufwärts des aktiven SCR angeordnet ist, wobei das Verfahren umfasst:
einen sechsten Schritt des Analysierens eines durch den zweiten NOₓ-Sensor (NOₓ(2)) erzeugten zweiten Signals im Frequenzbereich, um
a) das erste vorgegebene Frequenzband des zweiten Signals zu isolieren und zu prüfen, dass eine zweite Intensität (H') des zweiten Signals einen dritten vorgegebenen Schwellenwert (Th3) übersteigt, und/oder
b) ein zweites harmonisches Frequenzband mit einer größten relativen Intensität, die als die zweite Intensität (H') bezeichnet wird, auszuwählen, und
einen siebten Schritt des Erzwingens, dass das erste Band mit dem zweiten Frequenzband übereinstimmt.

3. Verfahren nach Anspruch 2, das ferner einen achten Schritt (CK3) des Prüfens, dass die zweite Intensität (H') des zweiten Bandes einen dritten vorgegebenen Schwellenwert (Th3) übersteigt, umfasst, wobei dann, wenn ja (CK3 = ja), der siebente Schritt ausgeführt wird, andernfalls das vorher festgelegte erste vorgegebene Band unverändert gelassen wird.

4. Verfahren nach Anspruch 3, wobei der aktive SCR-Katalysator in einem Abgasnachbehandlungssystem (ATS) einer Brennkraftmaschine enthalten ist und wobei dann, wenn die zweite Intensität (H') kleiner als der dritte vorgegebene Schwellenwert (Th3) ist, das Verfahren einen dritten Schritt (3) des Erzwingens vorsieht, dass die Brennkraftmaschine zeitlich wellige NOₓ-Emissionen erzeugt.

5. Verfahren nach einem der vorhergehenden Ansprüche, wobei der aktive SCR-Katalysator in einem Abgasnachbehandlungssystem (ATS) einer Brennkraftmaschine enthalten ist und wobei dann, wenn der Intensitätswert (H) kleiner als ein erster vorgegebener Schwellenwert ist, das Verfahren einen dritten Schritt (3) des Erzwingens vorsieht, dass die Brennkraftmaschine zeitlich wellige NOₓ-Emissionen erzeugt.

6. Verfahren nach Anspruch 4 oder 5, wobei angenommen wird, dass das Signal einen Messwert von NH₃ angibt, wenn nach dem dritten Schritt der Intensitätswert des ersten Frequenzbands kleiner als der zweite vorgegebene Schwellenwert ist.

7. Steuerverfahren eines aktiven SCR, das einen ersten Schritt des Ausführens aller Schritte nach einem der vorhergehenden Ansprüche 1 bis 6 umfasst und ferner einen weiteren Schritt (Wiederherstellung) des Ausführens einer Wiederherstellungsprozedur umfasst, wenn angenommen wird, dass das NOₓ-Sensorsignal einen Messwert von NH3 angibt.

8. Verfahren nach Anspruch 7, wobei die Wiederherstellungsprozedur eine plötzliche Verringerung der Einspritzung eines auf Harnstoff basierenden Reduktionsmittels in den aktiven SCR und/oder eine Zunahme der NOₓ-Produktion durch die Brennkraftmaschine umfasst.

9. Verfahren nach einem der vorhergehenden Ansprüche, wobei das durch den NOₓ-Sensor erzeugte Signal und/oder das durch den zweiten NOₓ-Sensor erzeugte Signal vorbereitend hochpassgefiltert werden.

10. Steuersystem eines aktiven SCR, das einen NOₓ-Sensor, der stromabwärts eines aktiven SCR in Bezug auf ein Ausströmen eines Abgases, das durch den aktiven SCR strömt, angeordnet ist, und eine Verarbeitungseinheit, die konfiguriert ist, wenigstens alle Schritte nach einem der Ansprüche 1, 4-9 auszuführen, umfasst.

11. Brennkraftmaschine, die umfasst:
- ein Abgasnachbehandlungssystem (ATS), das einen aus einem SCR ausgebildeten aktiven SCR und eine Messeinheit zum Messen eines auf Harnstoff basierenden Reduktionsmittels, die stromaufwärts des SCR gemäß einer Ausströmrichtung des durch die Brennkraftmaschine erzeugten Abgases angeordnet ist, umfasst,
- einen ersten NOₓ-Sensor, der stromabwärts des aktiven SCR angeordnet ist,
- eine erste Verarbeitungseinheit (CPU1), die konfiguriert ist, eine Einspritzung des auf Harnstoff basierenden Reduktionsmittels auf der Grundlage einer durch den ersten NOₓ-Sensor ausgeführten Messung des NOₓ zu steuern,
- eine zweite Verarbeitungseinheit (CPU2), die konfiguriert ist, alle Schritte nach einem der Ansprüche 1, 4-9 auszuführen.

12. Kraftmaschine nach Anspruch 11, wobei die zweite Verarbeitungseinheit konfiguriert ist, zu der ersten Verarbeitungseinheit zu übertragen, ob angenommen wird, dass das durch den ersten Sensor erzeugte Signal einen Messwert des NOₓ und/oder des NH3 angibt.

13. Kraftmaschine nach einem der Ansprüche 11 oder 12, wobei das ATS ferner mit einem zweiten NOₓ-Sensor, der stromaufwärts des aktiven SCR angeordnet ist, versehen ist und wobei die zweite Verarbeitungseinheit konfiguriert ist, ferner außerdem alle Schritte nach einem der Ansprüche 3 oder 4 auszuführen.

## Revendications

1. Procédé d'analyse d'un signal généré par un capteur de NOx agencé en aval d'un catalyseur de RCS active, le procédé comprenant :
- une première étape d'acquisition (1) du signal généré par le capteur de NOx,
- une deuxième étape consécutive (2) comprenant :
a) l'isolation d'une première bande de fréquences prédéterminée dudit signal, et
b) le calcul d'une valeur d'intensité (H) dudit signal dans ladite première bande de fréquences,
**caractérisé en ce qu'**il comprend une troisième étape consécutive (CK1) de comparaison de ladite valeur d'intensité avec un premier seuil prédéterminé (Th1) ; ledit signal étant jugé comme indicatif d'une mesure de NOx lorsque ladite valeur d'intensité est supérieure ou égale (H >= Th1) audit premier seuil prédéterminé ; dans lequel, lorsque ladite valeur d'intensité est inférieure (H < Th1) audit premier seuil prédéterminé, le procédé comprend une cinquième étape consécutive (CK2) de comparaison de ladite valeur d'intensité avec un deuxième seuil prédéterminé (Th2), inférieur ou égal audit premier seuil prédéterminé (Th1) ; ledit signal étant jugé comme indicatif d'une mesure de NH3 lorsque ladite valeur d'intensité est inférieure audit deuxième seuil prédéterminé (H < Th2).

2. Procédé selon la revendication 1, dans lequel ladite RCS active est incluse dans un système de post-traitement (ATS) des gaz d'échappement comprenant un deuxième capteur de NOx agencé en amont de ladite RCS active, le procédé comprenant une sixième étape d'analyse d'un deuxième signal généré par ledit deuxième capteur de NOx (NOx(2)) dans le domaine fréquentiel, pour
a) isoler ladite première bande de fréquences prédéterminée dudit deuxième signal et vérifier qu'une deuxième intensité (H') dudit deuxième signal, dépasse un troisième seuil prédéterminé (Th3) et/ou
b) sélectionner une deuxième bande de fréquences harmoniques ayant la plus grande intensité relative, appelée deuxième intensité (H'), et une septième étape forçant ladite première bande à coïncider avec ladite deuxième bande de fréquences.

3. Procédé selon la revendication 2, comprenant en outre une huitième étape (CK3) vérifiant que ladite deuxième intensité (H') de ladite deuxième bande dépasse un troisième seuil prédéterminé (Th3), et si oui (CK3 = oui), ladite septième étape est effectuée, sinon ladite première bande prédéterminée précédemment définie est laissée inchangée.

4. Procédé selon la revendication 3, dans lequel ledit catalyseur de SCR active est inclus dans un système de post-traitement (ATS) des gaz d'échappement d'un moteur à combustion interne, et dans lequel, lorsque ladite deuxième intensité (H') est inférieure audit troisième seuil prédéterminé (Th3), le procédé prévoit une troisième étape (3) forçant ledit moteur à combustion interne à produire des émissions de NOx ondulant dans le temps.

5. Procédé selon l'une quelconque des revendications précédentes, dans lequel ledit catalyseur de RCS active est inclus dans un système de post-traitement (ATS) des gaz d'échappement d'un moteur à combustion interne, et dans lequel, lorsque ladite valeur d'intensité (H) est inférieure audit premier seuil prédéterminé, le procédé prévoit une troisième étape (3) forçant ledit moteur à combustion interne à produire des émissions de NOx ondulant dans le temps.

6. Procédé selon la revendication 4 ou 5, dans lequel ledit signal est jugé comme indicatif d'une mesure de NH3 lorsque, à la suite de ladite troisième étape, ladite valeur d'intensité de ladite première bande de fréquences est inférieure audit deuxième seuil prédéterminé.

7. Procédé de commande de RCS active comprenant une première étape effectuant toutes les étapes de l'une quelconque des revendications précédentes 1 à 6 et comprenant en outre une étape supplémentaire (récupération) effectuant une procédure de récupération lorsque ledit signal de capteur de NOx est jugé comme indicatif d'une mesure de NH3.

8. Procédé selon la revendication 7, dans lequel ladite procédure de récupération comprend une réduction soudaine de l'injection d'un agent réducteur à base d'urée dans ladite RCS active et/ou une augmentation de la production de NOx par le moteur à combustion interne.

9. Procédé selon l'une quelconque des revendications précédentes, dans lequel ledit signal généré par ledit capteur de NOx et/ou ledit signal généré par ledit deuxième capteur de NOx est préalablement filtré en passe-haut.

10. Système de commande de RCS active comprenant un capteur de NOx agencé en aval d'une RCS active, en rapport avec un écoulement à la sortie des gaz d'échappement passant par ladite RCS active, et une unité de traitement configurée au moins pour effectuer toutes les étapes de l'une quelconque des revendications 1, 4 à 9.

11. Moteur à combustion interne comprenant :
- un système de post-traitement (ATS) des gaz d'échappement comprenant une RCS active formée d'une RCS et d'une unité de dosage pour doser un agent réducteur à base d'urée agencé en amont de la RCS, selon une direction d'écoulement à la sortie des gaz d'échappement produits par le moteur à combustion interne,
- un premier capteur de NOx agencé en aval de ladite RCS active,
- une première unité de traitement (CPU1) configurée pour commander une injection dudit agent réducteur à base d'urée sur la base d'une mesure de NOx effectuée par ledit premier capteur de NOx,
- une deuxième unité de traitement (CPU2) configurée pour effectuer toutes les étapes de l'une quelconque des revendications 1, 4 à 9.

12. Moteur selon la revendication 11, dans lequel ladite deuxième unité de traitement est configurée pour notifier à ladite première unité de traitement si ledit signal généré par ledit premier capteur est jugé comme indicatif d'une mesure de NOx et/ou de NH3.

13. Moteur selon l'une des revendications 11 ou 12, dans lequel ledit ATS est en outre pourvu d'un deuxième capteur de NOx agencé en amont de ladite RCS active, et dans lequel ladite deuxième unité de traitement est configurée pour, également, effectuer en outre toutes les étapes de l'une quelconque des revendications 3 ou 4.
